# EUROPÄISCHE PATENTSCHRIFT

(11) **EP 1 435 983 B1**
(45) Veröffentlichungstag und Bekanntmachung des Hinweises auf die Patenterteilung: **28.12.2005**
(21) Anmeldenummer: 02801318.3
(22) Anmeldetag: 08.10.2002
(51) Int. Cl.: A61K 35/78, A23L 1/30

(54) **KONZENTRAT, UMFASSEND GRÜNTEE, TRAUBENSCHALENEXTRAKT UND TRAUBENKERNEXTRAKT, SEINE HERSTELLUNG UND VERWENDUNG**
CONCENTRATE COMPRISING GREEN TEA, GRAPE SKIN EXTRACT AND GRAPE SEED EXTRACT, THE PRODUCTION THEREOF AND THE USE OF THE SAME
CONCENTRE COMPOSE DE THE VERT, D'EXTRAIT DE PEAU DE RAISIN ET D'EXTRAIT DE GRAIN DE RAISIN, FABRICATION ET UTILISATION

(30) Priorität: 15.10.2001 DE 10150824
(43) Veröffentlichungstag der Anmeldung: 14.07.2004
(73) Patentinhaber: Capri Sun AG, 6300 Zug (CH)
(72) Erfinder: WILD, Hans-Peter, 69214 Eppelheim (DE); SASS, Matthias, 68723 Oftersheim (DE)
(74) Vertreter: Grünecker, Kinkeldey, Stockmair & Schwanhäusser Anwaltssozietät
(86) Internationale Anmeldenummer: PCT/EP2002/011251
(87) Internationale Veröffentlichungsnummer: WO 2003/033005

(56) Entgegenhaltungen:
- US-A- 5 744 187
- US-A- 5 904 924
- BODYWISE, [Online] XP002222675 Gefunden im Internet: <URL:http://www.bodywise.com/bwcatalog/PAS 119E.asp> [gefunden am 2002-11-27]
- DR. MYATT'S, [Online] XP002222676 Gefunden im Internet: <URL:http://www.drmyattswellnessclub.com/m yattswellnessclub9.html> [gefunden am 2002-11-27]

## Beschreibung

Die vorliegende Erfindung betrifft ein Konzentrat aus pflanzlichen Extrakten, ein Verfahren zu seiner Herstellung sowie seine Verwendung.

In der menschlichen Ernährung werden seit vielen Jahrhunderten gesundheitlich relevante Wirkstoffe aufgenommen. Forschungen in jüngerer Zeit weisen auf präventiv wirksame Effekte von in Lebensmitteln enthaltenen sekundären Pflanzenstoffen hin. Einen guten Überblick dazu bietet B. Watzl, C. Leitzmann, *Bioaktive Substanzen in Lebensmitteln*, 2. Auflage, 1999, Hippokrates Verlag, Stuttgart.

Die grösste Gruppe innerhalb dieser sekundären Pflanzenstoffe sind die Polyphenole. Der Begriff Polyphenole stellt eine Sammelbezeichnung für aromatische Verbindungen dar, die mindestens zwei phenolische Hydroxygruppen im Molekül enthalten (Römpp Lexikon Chemie, 10. Auflage, 1999, Georg Thieme Verlag Stuttgart, S. 3491). Extrakte von Früchten, Kräutern, Gemüse und weiteren Pflanzen mit einem hohen Gehalt an phenolischen Komponenten sind von wachsendem Interesse für die Lebensmittelindustrie, da sie einen antioxidativen Charakter und somit einen gesundheitlichen positiven Effekt haben.

Grüntee bzw. Grüntee-Extrakte weisen einen hohen Gehalt an Polyphenolen auf und werden daher bevorzugt für die Herstellung von Getränken mit gesundheitlichen Potential eingesetzt.

Grüner Tee ist fast geruchlos, schmeckt adstringierend und angenehm bitter (vgl. Hagers Handbuch der Pharmazeutischen Praxis, Band 4, 5. Auflage, 1992, Springer-Verlag, Berlin Heidelberg, S. 628 ff). Der bittere Geschmack wird durch die im Tee enthaltenen Polyphenole hervorgerufen. Gefunden werden im grünen Tee hauptsächlich Catechine, Flavonolglykoside, Bisflavonole und Chlorogensäure. Die kleinmolekularen Komponenten dieser Polyphenole, insbesondere die Dimeren, haben kapillarabdichtende und antiinflammatorische Wirkungen.

Neben seiner gesundheitlichen Wirkung wird Grüner Tee auch als geistiges Stimulans geschätzt; er steigert die Konzentration und fördert die Ausdauer.

Weitere Substanzen mit hohem Polyphenolgehalt sind Traubenschalen- und Traubenkernextrakte. Hierbei ist insbesondere der Gehalt an oligomeren Procyanidinen in Traubenkernextrakten hervorzuheben, einer Untergruppe der flavonoiden Polyphenole. Procyanidine sind aufgrund ihrer chemischen Struktur wasserlöslich und besitzen ein niedriges Molekulargewicht (MG < 7000), welches sie hochgradig bioverfügbar macht. Physiologisch wirken Procyanidine blutdrucksenkend und antiatherosklerot und sie gelten als sehr effektive Radikalfänger, worauf ihre antioxidative Wirkung beruht (Römpp Lexikon Chemie, Naturstoffe, 1997, Georg Thieme Verlag Stuttgart, S. 514).

Im Zellsaft zahlreicher Blüten und Früchten kommen Anthocyanidine vor, die neben ihrer Farbwirkung auch ein antioxidatives Potential aufweisen. Anthocyanidine leiten sich von den Anthocyanen ab, deren Aglykone sie darstellen. Zu den wichtigsten Anthocyanidinen zählen Pelargonidin, Cyanidin, Delphinidin, Päonidin, Petunidin und Malvidin. Das Malvidin-3-glucosid ist beispielsweise in Heidelbeeren oder in den Schalen von roten Trauben enthalten. Anthocyanidine werden ebenfalls zur Gruppe der Polyphenole gezählt.

Ferner werden in der Nahrungsmittelindustrie auch Extrakte aus Pilzen oder Früchten eingesetzt, die ebenfalls gesundheitlich positive Wirkungen besitzen. So zeigen beispielsweise Extrakte des asiatischen Shütake-Pilzes immunstimulierende Wirkungen. Fruchtextrakte aus beispielsweise Papaya oder Ananas sind reich an natürlichen Enzymen wie Bromelain oder Papain, die anregend auf die Verdauung wirken sollen.

Allerdings konnte nachgewiesen werden, dass mit steigendem antioxidativen Potential auch die Bitterkeit der einzelnen Rohstoffe zunimmt. Dies entspricht auch den Erwartungen, da das antioxidative Potential zumeist in den phenolischen Inhaltsstoffen der Rohwaren begründet ist und diese phenolischen Komponenten deutlich bitter schmecken. Für die Produktentwicklung in der Lebensmittelindustrie bedeutet dies jedoch, dass der Bittergeschmack mit steigendem gesundheitlichem Potential zunimmt, ein für die Akzeptanz durch den Verbraucher nicht erwünschter Effekt.

US-A-5,904,924 beschreibt eine pulvrige Zusammensetzung, welche eine Mischung aus pflanzlichen Nährstoffen, hochwirksamen Kräutern, Phytochemikalien, α-Aminosäuren, Grünalgen, Blaualgen, Pulvern aus grünen Grassäften und Vitaminen der B-Gruppe in bestimmten Mengenverhältnissen enthält. Die Zusammensetzung ist als ein Nahrungsergänzungsmittel gedacht, das dem Anwender nach der Einnahme einen Energieschub versetzt und allgemein zum Wohlbefinden beiträgt.

Aufgabe der vorliegenden Erfindung ist es daher, ein Konzentrat zur Verfügung zu stellen, das ein hohes antioxidatives Potential aufweist bei gleichzeitig reduzierter Bitterkeit und somit eine hohe sensorische Akzeptanz beim Verbraucher erzeugt.

Diese Aufgabe wird erfindungsgemäß gelöst durch ein Konzentrat, umfassend
5,0 Gew.-% bis 20,0 Gew.-% Grüntee,
10,0 Gew.-% bis 60,0 Gew.-% Traubenschalenextrakt und
0,5 Gew.-% bis 5,0 Gew.-% Traubenkernextrakt,
jeweils bezogen auf die Gesamtmenge des Konzentrats.

Die vorliegende Erfindung beinhaltet weiterhin ein Verfahren zur Herstellung dieses Konzentrats sowie die Verwendung des Konzentrats in einem Lebensmittel, insbesondere in einem Getränk.

Die Herstellung der Extrakte kann durch übliche Verfahren erfolgen. Es ist auch möglich, kommerziell erhältliche Extrakte einzusetzen.

Das erfindungsgemäße Konzentrat enthält Grüntee, in einer bevorzugten Ausführungsform einen Grüntee-Extrakt. Der Tee-Extrakt wird vorzugsweise durch eine wäßrige Extraktion der getrockneten Teeblätter erhalten, die bei erhöhter Temperatur, bevorzugt bei einer Temperatur von 10°C bis 90°C, ganz besonders bevorzugt von 10°C bis 60°C durchgeführt wird.

Die im erfindungsgemäßen Konzentrat enthaltenen Traubenschalenextrakte werden bevorzugt aus den Schalen roter Trauben hergestellt. Diese Extrakte werden zum Beispiel aus dem Trester der betreffenden Trauben durch wäßrige Extraktion erhalten. Vorzugsweise werden Extrakte zugefügt, die Malvidin-3-glycosid in einer Menge von 0,05 Gew.-% bis 10,0 Gew.-%, bevorzugt von 0,1 Gew.% bis 4,0 Gew.-%, ganz besonders bevorzugt von 0,4 Gew.-% bis 1,0 Gew.-% enthalten.

Ferner enthält das erfindungsgemäße Konzentrat Traubenkernextrakte, bevorzugt Extrakte mit einem Gehalt an oligomeren Procyanidinen zwischen 20 Gew.-% und 95 Gew.-%, besonders bevorzugt zwischen 20 Gew.-% und 60 Gew.-%. In einem bevorzugten Verfahren zur Herstellung der Extrakte werden Traubenkerne gemahlen und das Homogenisat mit Wasser oder Wasser/Ethanol-Mischungen extrahiert.

Des weiteren enthält das erfindungsgemäße Konzentrat Extrakte aus essbaren Pilzen, wie zum Beispiel Shiitake (Lentinus edode), Maitake (Grifola Frondosa) oder Reishi (Lucid Ganoderma). Eine Extraktion der Pilze kann folgendermassen durchgeführt werden: Frische Pilze werden luftgetrocknet und anschließend gemahlen. Mit Wasser als Lösungsmittel wird die Pilzmaische extrahiert und der gewonnene Extrakt anschließend getrocknet.

In einer bevorzugten Ausführung können dem erfindungsgemäßen Konzentrat Extrakte aus Früchten zugesetzt werden. Beispiele für verwendbare Früchte sind Papaya, Ananas, Aprikose, Mango, Pfirsich, Banane. Vorzugsweise werden Papaya- und/oder Ananasextrakte zugefügt. In einem bevorzugten Verfahren zur Herstellung eines Fruchtextraktes werden die Früchte mechanisch zerkleinert, bis sie eine breiförmige Konsistenz aufweisen. Diese Mischung wird mittels einer Fruchtpresse ausgepresst und anschließend filtriert. Der erhaltene Fruchtsaft kann bei Bedarf nach herkömmlichen Methoden auf eine gewünschte Konzentration eingedickt werden.

Weiterhin können dem erfindungsgemäßen Konzentrat Vitamine zugefügt werden. Beispiele umfassen wasserlösliche Vitamine, wie Vitamin B₁, Vitamin B₂, Vitamin B₆, Vitamin B₁₂, Vitamin C, Vitamin H (Biotin), Nicain, Pantothensäure oder Folsäure und fettlösliche Vitamine, wie Provitamin A (beta-Carotin), Vitamin E, Vitamin K, Vitamin D. Besonders bevorzugt ist Vitamin C. Die Vitamine werden dem erfindungsgemäßen Konzentrat vorzugsweise in Pulverform oder, bei fettlöslichen Vitaminen, in einer wasserdispergierbaren Form zugefügt. Die üblichen Mengen liegen zwischen 0,1 Gew.-% und 10,0 Gew.-%, bevorzugt zwischen 0,5 Gew.-% und 3,5 Gew.-%.

Ferner können dem erfindungsgemäßen Konzentrat Mineralstoffe und/oder Spurenelemente, wie Calcium, Magnesium, Zink, Selen, Eisen, Chrom zugesetzt werden. Bevorzugt hiervon ist Seien. Die Menge an in dem erfindungsgemäßen Konzentrat enthaltenen Mineralstoffen hängt von der Art des Mineralstoffs und dem Bedarf dafür ab. Sie liegt im allgemeinen im Bereich von 0,1 bis 10 Gew.-%, vorzugsweise im Bereich von 0,2 bis 8 Gew.-%.

Außerdem kann das erfindungsgemäße Konzentrat weiterhin mindestens ein Aroma, vorzugsweise eine Aromakombination, enthalten. Als Aromastoffe können natürliche, naturidentische und/oder künstliche Aromastoffe verwendet werden. Vorzugsweise kommen natürliche Aromen in Betracht. Das Aroma bzw. die Aromakombination führt vorzugsweise zu einem fruchtigen, frischen oder exotischen Geschmack des aus dem erfindungsgemäßen Konzentrat erhaltenen Getränks oder sonstigem Lebensmittel. Übliche Mengen der Aromen bzw. der Aromakombination betragen 0,1 bis 5 Gew.-%, vorzugsweise 0,5 bis 3,5 Gew.-%.

Dem erfindungsgemäßen Konzentrat können weitere, in Nahrungsmitteln gebräuchliche Additive zugesetzt werden. Diese Additive können beispielsweise Säuerungsmittel, wie Zitronensäure, Milchsäure, Äpfelsäure, Phosphorsäure, Stabilisatoren, wie Johannisbrotkernmehl, Pektin, Guarkernmehl, Verdickungsmittel, wie Xanthan, Gellan, Alginate, Gummi arabicum, Süßungsmittel, wie Fructose, Saccharose, Invertzucker, Honig oder Süßstoffe, wie Aspartam, Acesulfam K, Saccharin, Cyclamat, Neohesperidin, Thaumatin, Sucralose sein.

Der Anteil der Komponenten in dem erfindungsgemäßen Konzentrat liegt im Bereich von 5,0 Gew.-% bis 20,0 Gew.% für Grüntee-Extrakt von 10,0 Gew.-% bis 60,0 Gew.-% für Traubenschalenextrakt, von 0,5 Gew.-% bis 5,0 Gew.-% für Traubenkernextrakt und von 0,1 Gew.-% bis 5,0 Gew.-% für den Pilzextrakt (bezogen auf die Gesamtmenge).

Bevorzugt sind Mengenanteile von 10,0 Gew.-% bis 15,0 Gew.-% für Grüntee, von 40,0 Gew.-% bis 50,0 Gew.-% für Traubenschalenextrakt, von 1,0 Gew.-% bis 3,0 Gew.-% für Traubenkemextrakt und von 0,1 Gew.% bis 1,0 Gew.-% für den Pilzextrakt.

Zur Herstellung des erfindungsgemäßen Konzentrats werden die einzelnen Komponenten miteinander gemischt. Pulverförmige Rohstoffe werden vor der Zugabe in Wasser vollständig gelöst. Während der Zubereitung der Mischung wird diese fortwährend gerührt, um eine homogene Mischung zu erhalten. Zum Zwecke der Haltbarmachung kann das Konzentrat pasteurisiert werden.

Das erfindungsgemäße Konzentrat kann in flüssiger oder getrockneter Form vorliegen.

Um das Konzentrat in getrockneter Form zu erhalten, wird den gemischten Extrakten auf bekannte Weise Wasser entzogen, vorzugsweise durch Sprühtrocknung. Die Sprühtrocknung stellt eine effiziente Methode zur Umwandlung von Flüssigkeiten in Pulver dar. Damit ist es möglich, ein Pulver mit kontrollierten physikalischen Eigenschaften, wie Fließfähigkeit, Restwassergehalt oder Dichte, zu erhalten. Gleichzeitig können auch die Eigenschaften der darin enthaltenen Geschmackskomponente bezüglich Qualität oder Freisetzung kontrolliert werden (A. Nilesen, J. Getler in Flavourings, Wiley-VCH Verlag GmbH Weinheim, 1998, S. 88 ff).

Das erfindungsgemäße Konzentrat kann in üblicher Weise zu Tabletten, Granulaten oder Pulvern verarbeitet werden. Beispiele für erfindungsgemäße Tabletten umfassen nicht-überzogene Tabletten in Form von Pulverpreßlingen, einschließlich Brausetabletten, Filmtabletten und Dragees.

Das erfindungsgemäße Konzentrat kann zur Herstellung von Getränken, beispielsweise durch Verdünnung eines flüssigen Konzentrats oder durch Auflösen eines Pulvers, einer Tablette oder eines Granulats in Wasser oder kalten oder heißen Getränken, wie Fruchtsäften oder Tees, eingesetzt werden.

Weiterhin kann das erfindungsgemäße Konzentrat als Nahrungsergänzungsmittel anderen Lebensmitteln wie Müslizubereitungen, Milchprodukten, z.B. Joghurt, Quarkspeisen, Dauerbackwaren zugesetzt werden.

Die Erfindung wird anhand der folgenden Beispiele näher erläutert.

### Beispiel 1

Es wurden verschiedene Konzentrate in den in Tabelle 1 angegebenen Verhältnissen der Bestandteile hergestellt. Die im Konzentrat verwendeten Extrakte wurden auf die folgende Weise hergestellt:
Grüntee-Extrakt:
   Grob gemahlene Grünteeblätter werden mit der 3fachen Menge 60°C warmen Wasser vermischt und nach 30 Minuten Kontaktzeit werden die Blattreste durch Zentrifugation abgetrennt. Der erhaltene Extrakt wird mittels Dünnschichtverdampfung konzentriert bis auf eine Trockenmasse von 60%-mas.
Traubenschalenextrakt:
   Traubentrester werden in einen Extraktionstank gegeben in dem das Lösemittel (40°C warmes Wasser) im Gegenstromverfahren durch die Trester gepumpt wird. Der gewonnene Rohextrakt wird filtriert und anschließend auf eine Trockenmasse von 30%-mas konzentriert.
   Traubenkernextrakt.
   Traubenkerne werden gemahlen und in einem Extraktionstank im Gegenstrom mit einem Wasser/Ethanolgemisch bei 40°C extrahiert. Der Alkohol wird aus dem gewonnenen Extrakt durch Destillation entfernt und der Rückstand wird getrocknet.
Pilzextrakt:
   Getrocknete Pilze werden gemahlen und mit Wasser im Gegenstrom extrahiert. Die unlöslichen Bestandteile werden durch Zentrifugation abgetrennt und der gewonnene Extrakt wird zu einem Pulver getrocknet.

Der Gesamtphenolgehalt und das antioxidative Potential der Konzentrate wurden bestimmt. Die Bestimmung des Phenolgehalts erfolgte photometrisch nach folgender Literaturstelle:
Singelton, V-L; Rossi, J.A. Colorimetry of Total Phenolics with Phosphomolybdic- phosphotungstic Acid Reagents. Am. J. Enol. Vitic. 1965, 16, 144.

Der Gesamtphenolgehalt wird "berechnet als Gallussäure" und in mg/l angegeben.

Zur Probenvorbereitung werden die Proben auf einen Gesamtphenolgehalt (berechnet als Gallussäure) von 50-500mg/l verdünnt.

Das antioxidative Potential wurde nach der TEAC-Methode bestimmt. TEAC steht als Abkürzung für Trolox Equivalent Antioxidant Capacity und beschreibt ein Verfahren zur Bestimmung der antioxidativen Kapazität einer Substanz in-vitro im Vergleich zu einem wasserlöslichen Vitamin-E-Equivalent ("Trolox"). Das Verfahren wird beispielsweise in folgender Literaturstelle beschrieben:
Re, R.; Pellegrini, N.; Proteggente, A.; Yang, M.; Rice-Evans, C. Antioxidant Activity Applying an Improved Abts Radical Cation Decoloratization Assay. Free Radical Biology & Medicine 1999, 26, 1231.

Die Ergebnisse sind in Tabelle 1 zusammengestellt.

**Tabelle 1**

| | Grüntee-Extrakt | Traubenschalenextrakt | Traubenkernextrakt | Pilzextrakt | Vitamin C | Gesamtphenole [mg/l] | Ergebnis "antioxidativ" TEAC |
|---|---|---|---|---|---|---|---|
| 1 | 3,0 g/l | 0 | 0 | 0 | 0 | 540 | 9,15 |
| 2 | 0 | 12,0 g/l | 0 | 0 | 0 | 300 | 4,44 |
| 3 | 0 | 0 | 0,5 g/l | 0 | 0 | 315 | 3,34 |
| 4 | 0 | 0 | 0 | 0,1 g/l | 0 | 0 | 0 |
| 5 | 0 | 0 | 0 | 0 | 0,3 g/l | 0 | 1,47 |
| 6 | 3,0 g/l | 12,0 g/l | 0 | 0 | 0 | 840 | 13,6 |
| 7 | 3,0 g/l | 0 | 0,5 g/l | 0 | 0 | 855 | 12,5 |
| 8 | 0 | 12,0 g/l | 0,5 g/l | 0 | 0 | 615 | 7,8 |
| 9 | 0 | 12,0 g/l | 0,5 g/l | 0,1 g/l | 0,3 g/l | 615 | 9,3 |
| 10 | 3,0 g/l | 12,0 g/l | 0,5 g/l | 0,1 g/l | 0,3 g/l | 1155 | Theo: 18,4 Real: 18,0 |

### Beispiel 2

Anhand von sensorischen Tests wurde die Bitterkeit verschiedener Extraktkombinationen bestimmt. Die Herstellung der verwendeten Extrakte erfolgte wie in Beispiel 1 beschrieben. Die Extrakte wurden miteinander gemischt und mit einer standardisierten Mischung aus Invertzuckersirup und Zitronensäure zu einem Getränk ausgemischt. Für den sensorischen Vergleich wurden alle Getränke auf das gleiche Verhältnis von Süße und Säure eingestellt, so daß nur die Bitterkeit als Beurteilungskriterium vom Verkoster bewertet wird.

Mit Hilfe von trainierten Verkostern wird die Bitterkeit der einzelnen Extrakte auf kontinuierlichen Skalen beurteilt. Die gewonnenen Meßdaten werden in einem Computer erfasst und mit Hilfe statistischer Methoden auf signifikante Unterschiede ausgewertet.

In Tabelle 2 ist der Gesamtphenolgehalt und das sensorische Ergebnis von Mischungen unterschiedlicher Zusammensetzung auf Basis einer Zucker/Säure-Mischung ohne Zusatz von Kohlensäure wiedergegeben.

**Tabelle 2**

| | Traubenschalenextrakt | Grüntee-Extrakt | Traubenkernextrakt | Gesamtphenolge halt [mg/l] | Bitterkeit |
|---|---|---|---|---|---|
| A | 12,0 g/l | 3,0 g/l | 0,5 g/l | 1120 | O |
| B | 12,0 g/l | 4,8 g/l | 0,0 g/l | 1120 | ++ |
| C | 12,0 g/l | 0,0 g/l | 1,37 g/l | 1120 | ++ |
| D | 44,8 g/l | 0,0 g/l | 0,0 g/l | 1120 | - |
| **Legende** ○ = Bewertung der Nullprobe, Referenzwert ++ = signifikant bitterer + = bitterer, aber nicht signifikant - = weniger bitter, aber nicht signifikant -- = signifikant weniger bitter | | | | | |

Zusammensetzungen, die alle drei Extrakte (Gemisch A) enthalten, wiesen bei einem Geschmackstest nur noch eine geringe Bitterkeit auf. Eine Ausmischung, die lediglich aus Grapeskinextrakt bestand wurde als etwas weniger bitter beschrieben, jedoch ist der TEAC-Wert dieser Komponente niedriger als der der anderen Rohstoffe, so daß kein hochantioxdatives Getränk erhalten werden kann. Sensorisch konnte kein signifikanter Unterschied ermittelt werden, daher muss die gleiche Bitterkeit attestiert werden. Demzufolge ist nur die Gesamtmischung aller Extrakte bei hohem antioxidativen Potential von geringer Bitterkeit.

Durch die sensorischen Tests konnte gezeigt werden, dass ein hoch-antioxidatives Konzentrat durch Mischen der oben genannte Rohstoffe bzw. Extrakte entwickelt werden kann, das trotz hohem Gesamtphenolgehalt eine geringe, für den Verbraucher akzeptable, Bitterkeit aufweist. Offenbar, und für den Fachmann überraschend, heben sich die Bitterwirkungen der einzelnen Komponenten gegenseitig auf.

## Patentansprüche

1. Konzentrat, umfassend
5,0 Gew.-% bis 20,0 Gew.-% Grüntee,
10,0 Gew.-% bis 60,0 Gew.-% Traubenschalenextrakt und
0,5 Gew.-% bis 5,0 Gew.-% Traubenkemextrakt,
jeweils bezogen auf die Gesamtmenge des Konzentrats.

2. Konzentrat nach Anspruch 1, **dadurch gekennzeichnet, dass** es weiterhin Pilzextrakt enthält.

3. Konzentrat nach Anspruch 2, **dadurch gekennzeichnet, dass** der Pilzextrakt ein Extrakt aus Shiitakepilzen ist.

4. Konzentrat nach einem der Ansprüche 1 bis 3, **dadurch gekennzeichnet, dass** es weiterhin Papaya- und/oder Ananasextrakt enthält.

5. Konzentrat nach einem der Ansprüche 1 bis 4, **dadurch gekennzeichnet, dass** es weiterhin Vitamin C enthält.

6. Konzentrat nach einem der Ansprüche 1 bis 5, **dadurch gekennzeichnet, dass** der Traubenschalenextrakt einen Gehalt an Malvidin-3-glycosid von 0,1 Gew.-% bis 1,0 Gew.-% aufweist.

7. Konzentrat nach einem der Ansprüche 1 bis 6, **dadurch gekennzeichnet, dass** der Traubenkemextrakt einen Gehalt an oligomeren Procyanidinen von 20 Gew.-% bis 95 Gew.-% aufweist.

8. Verfahren zur Herstellung eines Konzentrats nach einem der Ansprüche 1 bis 7, umfassend die Schritte Mischen der Rohstoffe und Haltbarmachung durch Pasteurisation.

9. Verwendung eines Konzentrats nach einem der Ansprüche 1 bis 7 in Lebensmitteln, inbesondere in Erfrischungsgetränken.

## Claims

1. A concentrate, comprising
5.0 wt% to 20.0 wt% green-tea,
10.0 wt% to 60.0 wt% grape-skin extract, and
0.5 wt% to 5.0 wt% grape-seed extract
based on the total weight of the concentrate, respectively.

2. The concentrate as defined in claim 1, **characterized in that** it also contains a fungus extract.

3. The concentrate as defined in claim 2, **characterized in that** the fungus extract is an extract of shiitake mushrooms.

4. The concentrate as defined in any one of claims 1 to 3, **characterized in that** it also contains an extract of papaya and/or pineapple.

5. The concentrate as defined in any one of claims 1 to 4, **characterized in that** it also contains vitamin C.

6. The concentrate as defined in any one of claims 1 to 5, **characterized in that** the grape-skin extract has a content of malvidin-3-glycoside of from 0.1 wt% to 1.0 wt%.

7. The concentrate as defined in any one of claims 1 to 6, **characterized in that** the grape-seed extract has a content of oligomeric procyanidins of from 20 wt% to 95 wt%.

8. A process for the production of a concentrate as defined in any one of claims 1 to 7, comprising the steps of mixing the raw materials and preserving the mixture by pasteurization.

9. Use of a concentrate as defined in any one of claims 1 to 7 in food stuff, in particular in refreshing beverages.

## Revendications

1. Concentré comprenant :
de 5,0 % en poids à 20,0 % en poids de thé vert,
de 10,0 % en poids à 60,0 % en poids d'extrait de peau de raisin et
de 0,5 % en poids à 5,0 % en poids d'extrait de pépin de raisin,
respectivement par rapport à la quantité totale du concentré.

2. Concentré selon la revendication 1, **caractérisé en ce qu'**il contient en outre de l'extrait de champignon

3. Concentré selon la revendication 2, **caractérisé en ce que** l'extrait de champignon est un extrait de champignons Shiitake.

4. Concentré selon l'une des revendications 1 à 3, **caractérisé en ce qu'**il contient en outre de l'extrait de papaye et/ou d'ananas.

5. Concentré selon l'une des revendications 1 à 4, **caractérisé en ce qu'**il contient en outre de la vitamine C.

6. Concentré selon l'une des revendications 1 à 5, **caractérisé en ce que** l'extrait de peau de raisin présente une teneur en malvidine-3-glucoside allant de 0,1 % en poids à 1,0 % en poids.

7. Concentré selon l'une des revendications 1 à 6, **caractérisé en ce que** l'extrait de pépin de raisin présente une teneur en procyanidines oligomères allant de 20 % en poids à 95 % en poids.

8. Procédé pour la préparation d'un concentré selon l'une des revendications 1 à 7, comprenant les étapes de mélange des matières premières et de conservation par pasteurisation.

9. Utilisation d'un concentré selon l'une des revendications 1 à 7 dans des produits alimentaires, en particulier dans des boissons rafraîchissantes.
